(19) **Europäisches Patentamt**
**European Patent Office**
**Office européen des brevets**

(11) **EP 3 646 869 B1**

(12) # EUROPEAN PATENT SPECIFICATION

(45) Date of publication and mention
of the grant of the patent:
**06.08.2025 Bulletin 2025/32**

(21) Application number: **18825157.3**

(22) Date of filing: **29.06.2018**

(51) International Patent Classification (IPC):
*A61K 31/437* (2006.01)   *A61K 39/395* (2006.01)
*A61K 45/00* (2006.01)   *A61P 35/00* (2006.01)
*A61P 37/04* (2006.01)   *A61P 43/00* (2006.01)
*C07D 471/04* (2006.01)   *A61K 45/06* (2006.01)

(52) Cooperative Patent Classification (CPC):
(C-Sets available)
**A61K 45/06; A61K 31/437; A61K 39/395;**
**A61K 45/00; A61P 35/00; A61P 37/04;**
**A61P 43/00; C07D 471/04**            (Cont.)

(86) International application number:
**PCT/JP2018/024776**

(87) International publication number:
**WO 2019/004417 (03.01.2019 Gazette 2019/01)**

(54) **CHEMOTHERAPY FOR CANCER USING AZABICYCLO COMPOUND**

CHEMOTHERAPIE GEGEN KREBS MIT AZABICYCLOVERBINDUNG

CHIMIOTHÉRAPIE ANTICANCÉREUSE METTANT EN  UVRE UN COMPOSÉ AZABICYCO

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **30.06.2017 JP 2017128823**
**15.09.2017 JP 2017177390**
**24.05.2018 JP 2018099931**

(43) Date of publication of application:
**06.05.2020 Bulletin 2020/19**

(73) Proprietor: **Taiho Pharmaceutical Co., Ltd.**
**Chiyoda-ku, Tokyo 101-8444 (JP)**

(72) Inventor: **OHKUBO, Shuichi**
**Tokyo 101-0047 (JP)**

(74) Representative: **Wächtershäuser & Hartz**
**Patentanwaltspartnerschaft mbB**
**Weinstraße 8**
**80333 München (DE)**

(56) References cited:
**EP-A1- 3 053 578        WO-A1-2011/004610**
**WO-A1-2015/046498      WO-A1-2016/130502**

- **S. OHKUBO ET AL: "TAS-116, a Highly Selective**
**Inhibitor of Heat Shock Protein 90  and  ,**
**Demonstrates Potent Antitumor Activity and**
**Minimal Ocular Toxicity in Preclinical Models",**
**MOLECULAR CANCER THERAPEUTICS, vol. 14,**
**no. 1, 1 January 2015 (2015-01-01), US, pages 14 -**
**22, XP055506314, ISSN: 1535-7163, DOI: 10.1158/**
**1535-7163.MCT-14-0219**
- **PROIA DAVID A. ET AL: "Targeting Heat-Shock**
**Protein 90 (HSP90) as a Complementary Strategy**
**to Immune Checkpoint Blockade for Cancer**
**Therapy", CANCER IMMUNOLOGY RESEARCH,**
**vol. 3, no. 6, 6 May 2015 (2015-05-06), US, pages**
**583 - 589, XP055774633, ISSN: 2326-6066,**
**Retrieved from the Internet <URL:https://**
**cancerimmunolres.aacrjournals.org/content/**
**canimm/3/6/583.full.pdf?**
**casa_token=9leijzNIhhIAAAAA:-3T_f2omY2W**
**qoEyWHKnBewCrs4uY5I6Vj3AtGRZP5TVIKXU5**
**gedeXoz0tPaZSmj3Y5hIgB102dg> DOI: 10.1158/**
**2326-6066.CIR-15-0057**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- MBOFUNG RINA M. ET AL: "HSP90 inhibition enhances cancer immunotherapy by upregulating interferon response genes", NATURE COMMUNICATIONS, vol. 8, no. 1, 6 September 2017 (2017-09-06), XP055774521, Retrieved from the Internet <URL:http://www.nature.com/articles/s41467-017-00449-z> DOI: 10.1038/s41467-017-00449-z
- MBOFUNG RINA M ET AL: "Mechanistic Merging of Treatment Modalities", CANCER IMMUNOLOGY RESEARCH ABSTRACT B105, 1 November 2016 (2016-11-01), XP055774677, Retrieved from the Internet <URL:https://cancerimmunolres.aacrjournals.org/content/4/11_Supplement/B105> [retrieved on 20210210], DOI: 10.1158/2326-6066.IMM2016-B105Published
- MALL, CHRISTINE ET AL.: "Repeated PD-1/PD-L1 monoclonal antibody administration induces fatal xenogeneic hypersensitivity reactions in a murine model of breast cancer", ONCOIMMUNOLOGY, vol. 5, no. 2, 2016, pages eI075114/I - e1075114/12, XP055566284, ISSN: 2162-402X
- MBOFUNG, M. RINA ET AL.: "Inhibition of HSP90 enhances T cell -mediated antitumor immune responses through expression of interferon-alpha response Genes", CANCER RESEARCH, vol. 76, no. 14, 2016, pages 4360, XP009518575, DOI: 10.1158/1538-7445.AM2016-4360
- FABIO, DI ROMANO ET AL.: "Dihydropyrrole [2, 3-d] pyridine Derivatives as Novel Corticotropin-Releasing Factor-1 Antagonists: Mapping of the Receptor Binding Pocket by in Silico Docking Studies", J. MED. CHEM., vol. 51, 2008, pages 7273 - 7286, XP055043235, ISSN: 0022-2623

(52) Cooperative Patent Classification (CPC): (Cont.)

C-Sets
A61K 31/437, A61K 2300/00;
A61K 45/06, A61K 2300/00

**Description**

Technical Field

The present invention relates to an antitumor agent comprising an azabicyclo compound or a salt thereof in combination with an immune checkpoint molecule regulator and an antitumor effect enhancing agent.

Background Art

A group of proteins called molecular chaperons is multifunctional in such a way that they promote the formation of the functional structures of other proteins or maintain these structures, promote correct association, inhibit unnecessary aggregation, protect other proteins from degradation, and promote secretion (Non Patent Literature 1). HSP90 is a molecular chaperon as abundant as approximately 1 to 2% of all intracellular soluble proteins and is however unnecessary for the biosynthesis of the majority of polypeptides, unlike other chaperon proteins (Non Patent Literature 1). Signaling-related factors (e.g., ERBB1/EGFR, ERBB2/HER2, MET, IGF1R, KDR/VEGFR, FLT3, ZAP70, KIT, CHUK/IKK, BRAF, RAF1, SRC and AKT), cell cycle regulators (e.g., CDK4, CDK6, Cyclin D, PLK1 and BIRC5), and transcriptional regulators (e.g., HIF-1$\alpha$, p53, androgen receptor, estrogen receptor and progesterone receptor) are known as the main client proteins whose structure formation or stability is regulated by HSP90 through the interaction therebetween (Non Patent Literatures 2 and 3). HSP90 is deeply involved in cell proliferation or survival by maintaining the normal functions of these proteins. Furthermore, HSP90 is required for the normal functions of mutated or chimeric factors (e.g., BCR-ABL and NPM-ALK) which cause carcinogenesis or exacerbation of cancer. This indicates the importance of HSP90 particularly for processes such as carcinogenesis, cancer survival, growth, exacerbation and metastasis (Non Patent Literature 2).

The inhibition of the chaperon functions of HSP90 by specific inhibitors such as geldanamycin causes the inactivation, destabilization and degradation of the client proteins, resulting in induction of a halt in cell proliferation or apoptosis (Non Patent Literature 4). In terms of the physiological functions of HSP90, HSP90 inhibitors are characterized in that they can simultaneously inhibit a plurality of signaling pathways involved in cancer survival/growth. Thus, the HSP90 inhibitors can serve as drugs having extensive and effective anticancer activity. Moreover, from the findings that cancer cell-derived HSP90 has higher activity and higher affinity for ATP or inhibitors than those of normal cell-derived HSP90, it has been expected that the HSP90 inhibitors would serve as drugs having high cancer selectivity (Non Patent Literature 5).

On the other hand, the immunological system is an important mechanism for self-defense against various diseases caused by *in vivo* and *in vitro* factors. Deterioration of the functions of the immunological system has pathologically adverse effects such as development of infectious diseases by bacteria and viruses, development of tumors, and delay of recovery from injuries and diseases. Therefore, activation of the immunological system is very important for prevention and treatment of various diseases. As one of new methods for treating cancer, cancer immunotherapy is being developed.

Activation of adaptive immunological reaction is initiated by binding of an antigenic peptide-MHC complex to a T-cell receptor (TCR). Further, this binding is regulated by costimulation or coinhibition due to binding between the B7 family which is a costimulatory molecule and the CD28 family which is a receptor of the B7 family. That is, for T-cells to be activated in an antigen-specific manner, two characteristic signal transduction events are required, and T-cells which have not undergone costimulation from the B7 family and have undergone only antigen-stimulation are turned into a nonresponsive state (anergy), so that immunological tolerance is induced in the T-cells.

By taking advantage of this mechanism, cancer cells suppress activation of antigen-specific T-cells to escape from the immune surveillance, and continuously grow. Thus, for cancer treatment, it is considered effective that by enhancement of costimulation and blocking of coinhibition, an *in vivo* antitumor immune response is induced in a cancer patient to control immune escape of a tumor, and various cancer immunotherapies targeted at costimulatory molecules (stimulatory costimulatory molecules) or coinhibitory molecules (inhibitory costimulatory molecules) have been proposed (Patent Literature 6). For example, as an immune checkpoint molecule regulator for activating T-cells by inhibiting binding of PD-1 and ligands thereof (PD-L1 and PD-L2), nivolumab (human-type IgG4 monoclonal antibody against human PD-1) is used for malignant melanoma etc. (Patent Literature 1 and Non Patent Literature 7).

The azabicyclo compound below or a salt thereof is known as a HSP90 inhibitor (Patent Literature 2), and a combination of the HSP90 inhibitor and another antitumor agent has been heretofore reported (Patent Literature 3).

Non patent Literature 8 describes that the HSP90 inhibitor ganetespib enhances responses to cancer immunotherapy through increased expression of interferon response genes. Patent Literature 4 describes a combination therapy of HSP90 inhibitors and PD-1 inhibitors for treating cancer.

However, the HSP90 inhibitor and an immune checkpoint molecule regulator have not been used in combination yet. In addition, the immunostimulatory action of the HSP90 inhibitor is not known.

Citation List

Patent Literature

Patent Literature 1: International Publication No. WO2004/004771
Patent Literature 2: International Publication No. WO2011/004610
Patent Literature 3: International Publication No. WO2015/046498
Patent Literature 4: International Publication No. WO2016/130502

Non Patent Literature

Non Patent Literature 1: Nat. Rev. Cancer, 5: 761-772 (2005)
Non Patent Literature 2: TRENDS Mol. Med., 10: 283-290 (2004)
Non Patent Literature 3: Clin. Cancer Res., 15: 9-14 (2009)
Non Patent Literature 4: Curr. Opin. Pharmacol., 8: 370-374 (2008)
Non Patent Literature 5: Drug Resist. Updat., 12: 17-27 (2009)
Non Patent Literature 6: Nat. Rev. Cancer, 12: 252-264 (2012)
Non patent Literature 7: N. Engl. J. Med., 366: 2443-2454 (2012)
Non patent Literature 8: Mbofung Rina M ET AL: "Mechanistic Merging of Treatment Modalities", Cancer Immunology Research abstract B105, 1 November 2016

Summary of the Invention

Technical problem

An object of the present invention is to provide a novel cancer treatment method which exhibits a remarkably excellent antitumor effect with little side effects.

Solution to Problem

The present inventor has combined the azabicyclo compound defined below and an immune checkpoint molecule regulator, and has studied the antitumor effect of the combination. As a result, the present inventor has found that the antitumor effect is more remarkably enhanced without causing severe side effects as compared to a case where a single agent is used.
In addition, the present inventor has found that the azabicyclo compound has immunostimulatory action.
Specifically, according to the claims, the present invention provides:

1. An antitumor agent comprising azabicyclo compound or a salt thereof and an immune checkpoint molecule regulator which are administered in combination, wherein the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide;

wherein the immune checkpoint molecule regulator is at least one or more selected from the group consisting of a PD-1 pathway antagonist and a CTLA-4 pathway antagonist,
wherein the PD-1 pathway antagonist is at least one or more selected from the group consisting of an anti-PD-1 antibody and an anti-PD-L1 antibody, and
wherein the CTLA-4 pathway antagonist is an anti-CTLA-4 antibody.

2. The antitumor agent according to claim 1, wherein the anti-PD-1 antibody is at least one or more selected from the group consisting of nivolumab and pembrolizumab, and the anti-PD-L1 antibody is at least one or more selected from the group consisting of atezolizumab, durvalumab and avelumab.
3. The antitumor agent according to claim 1 or 2, wherein the anti-CTLA-4 antibody is at least one or more selected from the group consisting of ipilimumab and tremelimumab.
4. An agent that is

(i) an antitumor effect enhancing agent for use in enhancing the antitumor effect of an immune checkpoint molecule regulator in tumor treatment, comprising an azabicyclo compound or a salt thereof as an active ingredient, or

(ii) an antitumor agent for use in treating a cancer patient given an immune checkpoint molecule regulator, wherein both are administered in parallel or at an administration interval of 1 to 14 days, the antitumor agent comprising an azabicyclo compound or a salt thereof, or

(iii) an antitumor agent for use in treating a cancer patient given an azabicyclo compound or a salt thereof, wherein both are administered in parallel or at an administration interval of 1 to 14 days, the antitumor agent comprising an immune checkpoint molecule regulator;

wherein the azabicyclo compound is as defined in claim 1 and the immune checkpoint molecule regulator is as defined in any one of claims 1 to 3.

5. An antitumor agent comprising an azabicyclo compound or a salt thereof in combination with an immune checkpoint molecule regulator; wherein the azabicyclo compound is as defined in claim 1 and the immune checkpoint molecule regulator is as defined in any one of claims 1 to 3.

6. An azabicyclo compound or a salt thereof for use in treating a tumor by administration thereof in combination with an immune checkpoint molecule regulator, the azabicyclo compound being as defined in claim 1 and the immune checkpoint molecule regulator being as defined as in any one of claims 1 to 3.

7. An azabicyclo compound or a salt thereof for use

(i) in enhancing the antitumor effect of an immune checkpoint molecule regulator, or

(ii) in treating tumor in a cancer patient given an immune checkpoint molecule regulator in parallel or at an administration interval of 1 to 14 days,

the azabicyclo compound being as defined in claim 1 and the immune checkpoint molecule regulator is as defined in any one of claims 1 to 3.

8. An immune checkpoint molecule regulator for use in treating a tumor in a cancer patient given an azabicyclo compound or a salt thereof, wherein both are administered in parallel or at an administration interval of 1 to 14 days, the azabicyclo compound being as defined in claim 1 and the immune checkpoint molecule regulator is as defined in any one of claims 1 to 3.

9. A combination of an azabicyclo compound or a salt thereof and an immune checkpoint molecule regulator for use in treating a tumor, wherein both are administered in parallel or at an administration interval of 1 to 14 days, the azabicyclo compound being as defined in claim 1 and the immune checkpoint molecule regulator is as defined in any one of claims 1 to 3.

Advantageous Effects of Invention

The antitumor agent of the present invention makes it possible to perform cancer treatment with high antitumor effects (in particular, a tumor reducing effect and a tumor growth delaying effect (life extending effect)) while suppressing development of side-effects. Hence, the life of a cancer patient is extended.

Brief Description of Drawings

[Figure 1] Figure 1 shows the effect of a combination of Compound 1 and an antitumor mouse PD-1 antibody in a mouse model which has been transplanted with a mouse bowel cancer cell line MC38.

[Figure 2] Figure 2a shows the ratio of regulatory T-cells in T-cells in tumor infiltrating leucocytes, and Figure 2b shows the ratio of CD8 positive cells in tumor infiltrating leucocytes.

[Figure 3] Figure 3 shows the effect of a combination of Compound 1 and an antitumor mouse PD-L1 antibody in a mouse model which has been transplanted with a mouse bowel cancer cell line MC38.

[Figure 4] Figure 4 shows the effect of a combination of Compound 1 and an antitumor mouse CTLA-4 antibody in a mouse model which has been transplanted with a mouse bowel cancer cell line MC38.

Description of Embodiments

The present invention relates to an antitumor agent and an antitumor effect enhancing agent, comprising an azabicyclo compound and an immune checkpoint molecule regulator (in particular, anti-PD-1 antibody, anti-PD-L1 antibody or anti-CTLA-4 antibody), which are administered in combination; and use thereof.

In the present invention, the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide (hereinafter, referred to as Compound 1).

The salt of the azabicyclo compound according to the present invention is not particularly limited as long as it is a pharmaceutically acceptable salt, and examples thereof include acid addition salts with inorganic acids such as hydrochloric acid, hydrobromic acid, hydroiodic acid, sulfuric acid, nitric acid and phosphoric acid, or organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, paratoluenesulfonic acid and glutamic acid; salts with inorganic bases such as sodium, potassium, magnesium, calcium and aluminum, organic bases such as methylamine, ethylamine, meglumine and ethanolamine, or basic amino acids such as lysine, arginine and ornithine; and ammonium salts.

The azabicyclo compound according to the present invention or a salt thereof can be synthesized in accordance with, for example, the method described in International Publication No. WO2011/004610.

The immune checkpoint molecule regulator in the present invention directly acts on immune checkpoint molecules, so that an *in vivo* antitumor immune response is induced in a cancer patient to control immune escape of a tumor.

Examples of the immune checkpoint molecule regulator include substances which promote the functions of costimulatory molecules (stimulatory costimulatory molecules), or substances which suppress the functions of coinhibitory molecules (inhibitory costimulatory molecules). Examples of the immune checkpoint molecules include B7 family (B7-1, B7-2, PD-L1, PD-L2, etc.), CD28 family (CTLA-4, PD-1, etc.), TNF superfamily (4-1BBL and OX40L), TNF receptor superfamily (4-1BB and OX40) molecules, and for the immune checkpoint molecule regulator, a substance targeted at immune checkpoint molecules can be used. Examples of the substance include PD-1 pathway antagonists, ICOS pathway agonists, CTLA-4 pathway antagonists, CD28 pathway agonists, BTLA pathway antagonists and 4-1BB pathway agonists.

In the present invention, the immune checkpoint molecule regulator is one or more selected from the group consisting of a PD-1 pathway antagonist and a CTLA-4 pathway antagonist, preferably a PD-1 pathway antagonist from the viewpoint of suppressing side-effects.

The PD-1 pathway antagonist inhibits immunosuppressive signals from PD-1 expressed on T-cells, and PD-L1 or PD-L2 which is a ligand of PD-1. The PD-1 pathway antagonist is an anti-PD-1 antibody or an anti-PD-L1 antibody, preferably an anti-PD-1 antibody.

The CTLA-4 pathway antagonist inhibits immunosuppressive signals from CTLA-4 expressed on T-cells, and B7-1 (CD80) or B7-2 (CD86) which is a ligand of CTLA-4. The CTLA-4 pathway antagonist is an anti-CTLA-4 antibody.

In one of the aspects of the present invention, the immune checkpoint molecule regulator is preferably at least one or more selected from the group consisting of an anti-PD-1 antibody, an anti-PD-L1 antibody and an anti-CTLA-4 antibody.

Examples of the antibodies include immunoglobulins (IgA, IgD, IgE, IgG, IgM, IgY, etc.), Fab fragments, F(ab')$_2$ fragments, single-stranded antibody fragments (scFv), single domain antibodies, and Diabody (Nat. Rev. Immunol., 6:343-357, 2006), which include monoclonal antibodies or polyclonal antibodies such as human antibodies, humanized antibodies, chimeric antibodies, mouse antibodies, llama antibodies and chicken antibodies.

Humanized IgG monoclonal antibodies or human IgG monoclonal antibodies are preferable.

The anti-PD-1 antibody in the present invention may be nivolumab or pembrolizumab.

The anti-PD-L1 antibody in the present invention may be atezolizumab, durvalumab, or avelumab, preferably atezolizumab.

The anti-CTLA-4 antibody in the present invention may be ipilimumab or tremelimumab, preferably ipilimumab.

These antibodies can be normally produced by a known method for preparation of an antibody.

The anti-PD-1 antibody is or will be sold as nivolumab or pembrolizumab, the PD-L1 antibody is or will be sold as atezolizumab, durvalumab or avelumab, the CTLA-4 antibody is or will be sold as ipilimumab or tremelimumab, and the CTLA-4-1g is or will be sold as abatacept. It is also possible use to these products.

In the present invention, when two or more immune checkpoint molecule regulators are used, for example, the anti-PD-1 antibody and the anti-CTLA-4 antibody can be used in combination, or a bispecific antibody capable of being bound to both PD-1 and CTLA-4 can be used. Examples of the bispecific antibody XmAb20717 (PD-1 x CTLA-4).

In the present invention, the dose per dosing day of the azabicyclo compound or a salt thereof is preferably 50 to 200%, more preferably 87.5 to 112.5%, particularly preferably 100%, of the recommended dosage of the singly administered azabicyclo compound or salt thereof, from the viewpoint of the enhancing effect on the antitumor effect of the immune checkpoint molecule regulator by the azabicyclo compound. The recommended dosage in a human is preferably 80 to 320 mg/body/day, more preferably 140 to 180 mg/body/day, particularly preferably 160 mg/body/day.

In the present invention, the dose per dosing day of the immune checkpoint molecule regulator is preferably 50 to 100%, more preferably 100% of the recommended dosage of the singly administered immune checkpoint molecule regulator, from the viewpoint of the enhancing action on the antitumor effect of the immune checkpoint molecule regulator by the azabicyclo compound.

Specifically, the recommended dosage of singly administered nivolumab is 2 mg/kg (body weight) per administration or 3 mg/kg (body weight) per administration, a dose approved in Japan, and thus the dose per dosing day of nivolumab in the present invention is preferably 1 to 3 mg/kg (body weight) per administration, more preferably 2 mg/kg (body weight) per administration or 3 mg/kg (body weight) per administration.

The recommended dosage of singly administered pembrolizumab is 2 mg/kg (body weight) per administration or 200 mg per administration, a dose approved in Japan, and thus the dose per dosing day of pembrolizumab in the present invention is preferably 1 to 2 mg/kg (body weight) per administration or 100 to 200 mg per administration, more preferably 2 mg/kg (body weight) or 200 mg per administration.

The recommended dosage of singly administered atezolizumab is 1200 mg per administration, a dose approved in US, and thus the dose per dosing day of atezolizumab in the present invention is preferably 600 to 1200 mg per administration, more preferably 1200 mg per administration.

The recommended dosage of singly administered avelumab or durvalumab is 10 mg/kg (body weight) per administration, a dose approved in US, and thus the dose per dosing day of avelumab or durvalumab in the present invention is preferably 5 to 10 mg/kg (body weight) per administration, more preferably 10 mg/kg (body weight) per administration.

The recommended dosage of singly administered ipilimumab is 3 mg/kg (body weight) per administration, a dose approved in Japan, and thus the dose per dosing day of ipilimumab in the present invention is preferably 1.5 to 3 mg/kg (body weight) per administration, more preferably 3 mg/kg (body weight) per administration.

In the present invention, the "recommended dosage" is a dose which is determined through a clinical test etc. and at which a maximum therapeutic effect is produced while safe use can be ensured without development of severe side-effects. Specifically, the "recommended dosage" is a dosage approved, recommended or suggested by public organizations or corporations such as Pharmaceuticals and Medical Devices Agency (PMDA), Food and Drug Administration (FDA) and European Medicines Agency (EMA), and described in attached documents, interview forms, treatment guidelines or the like, preferably a dose approved by a public organization selected from the group consisting of PMDA, FDA and EMA.

The administration schedule of the antitumor agent of the present invention can be appropriately selected according to the type of cancer, the stage of disease, etc.

The administration schedule of the azabicyclo compound or a salt thereof is preferably an administration schedule in which continuous administration for 5 days and cessation of administration for 2 days is repeated, specifically an administration schedule in which administration for 3 weeks using an administration method including administration for 5 days, followed by cessation of administration for 2 days in one week is set to one cycle, and the cycle is repeated.

The administration schedule of nivolumab is preferably an administration schedule in which administration is performed at intervals of 2 or 3 weeks.

The administration schedule of pembrolizumab, atezolizumab or ipilimumab is preferably an administration schedule in which administration is performed at intervals of 3 weeks.

The number of doses per day of the antitumor agent of the present invention is appropriately selected according to the type of cancer, the stage of disease, etc.

The number of doses per day of the azabicyclo compound or a salt thereof is preferably 1 or 2, more preferably 1. The number of doses per day of nivolumab, pembrolizumab, atezolizumab or ipilimumab is preferably 1.

The order of administration of the azabicyclo compound or a salt thereof and the immune checkpoint molecule regulator in the present invention can be appropriately selected according to the type of cancer, the stage of disease, etc., and these agents may be administered in any order, or in parallel. Here, when both the agents are not administered in parallel, the administration interval between both the agents can be appropriately selected as long as an enhancing effect on the antitumor effect is exhibited, and the administration interval is preferably 1 to 14 days, more preferably 1 to 7 days, still more preferably 1 to 5 days, particularly preferably 1 to 3 days.

The tumor to be targeted in the present invention is not particularly limited as long as an enhancing effect on the antitumor effect is exhibited, and the tumor is preferably a tumor against which the azabicyclo compound represented by the general formula (I) or a salt thereof exhibits an antitumor effect, more preferably a malignant tumor in which Hsp90 is involved.

Specific examples of the cancer to be targeted by the present invention include head and neck cancer, digestive organ cancer (esophagus cancer, gastric cancer, duodenal cancer, liver cancer, biliary tract cancer (gallbladder cancer, cholangiocarcinoma, etc.), pancreatic cancer, small intestine cancer, bowel cancer (colorectal cancer, colon cancer, rectal cancer, etc.), gastrointestinal stromal tumor, etc.), lung cancer (non-small cell lung cancer and small cell lung cancer), breast cancer, ovarian cancer, uterine cancer (cervical cancer, endometrial cancer, etc.), kidney cancer, bladder cancer, prostatic cancer and skin cancer. Here, the cancers include not only primary lesions but also cancers metastasizing to other organs (such as liver). In particular, from the viewpoint of antitumor effect and side-effects, the cancer is preferably head and neck cancer, digestive organ cancer, lung cancer, breast cancer, kidney cancer or skin cancer, more preferably digestive organ cancer, lung cancer or breast cancer, still more preferably bowel cancer, gastric cancer, gastrointestinal stromal tumor, lung cancer or breast cancer, with bowel cancer being particularly preferable. The antitumor agent of the present invention may be used for postoperative adjuvant chemotherapy to be carried out for prevention of recurrence after surgical removal of tumor, or may be used for preoperative adjuvant chemotherapy to be carried out before surgical removal of tumor.

As shown in Examples below, the azabicyclo compound or a salt thereof has immunostimulatory action. As used herein, the "immunostimulatory action" means the action of activating immune cells to induce cell division and production of various cytokines. The azabicyclo compound or a salt thereof has the action of activating T-cells, in particular, among immune cells. Here, the action of activating T-cells means, for example, increase in ratio of CD8 positive cells in tumor infiltrating leucocytes and/or decrease in ratio of regulatory T-cells in T-cells in tumor infiltrating leucocytes.

Therefore, an immunostimulant may contain the azabicyclo compound or a salt thereof as an active ingredient; the azabicyclo compound or a salt thereof may be used for immunostimulation; and a method for immunostimulation may include the step of administering an effective amount of an azabicyclo compound or a salt thereof to a patient in need of immunostimulation. Examples of the immunostimulation target include humans and other mammals, e.g. monkeys, mice, rats, rabbits, dogs, cats, bovines, horses, pigs and sheep.

The immunostimulatory action of the azabicyclo compound or a salt thereof enables prevention and/or treatment of diseases which can be ameliorated by immunostimulation, such as various infectious diseases, immunodeficiency diseases, diseases caused by age-related decline in immune function and virus-related tumors.

Examples of the infectious diseases include parasitic infections (e.g. Trypanosoma protozoa, malaria protozoa and toxoplasma); bacterial infections (e.g. pneumococcus, tuberculosis bacteria, staphylococcus aureus, anthrax bacteria, cholera bacteria, mycoplasma and pylori bacteria (Helicobacter pylori); and viral infections (e.g. human T-cell leukemia virus (HTLV-1), human immunodeficiency virus (HIV), papillomavirus (HPV), Epstein-Barr virus (EBV), cytomegalovirus (CMV), influenza virus (FLU), hepatitis B virus (HBV), herpesvirus, hepatitis C virus (HCV), SARS coronavirus, MERS coronavirus, dengue virus, Ebola virus).

A pharmaceutical composition for preventing and/or treating an infectious disease by immunostimulation may include the azabicyclo compound or a salt thereof; the azabicyclo compound or a salt thereof may be used for preventing and/or treating an infectious disease by immunostimulation; and a method for preventing and/or treating an infectious disease in a patient in need of the prevention and/or treatment, by immunostimulation, may include administering to the patient an effective amount of the azabicyclo compound or a salt thereof.

The references to the methods of treatment of this description are to be interpreted as references to compounds or pharmaceutical compositions of the present invention for use in those methods.

Examples of the immunodeficiency diseases include congenital immunodeficiency diseases and acquired immunodeficiency diseases, particularly acquired immunodeficiency diseases caused by infection with human immunodeficiency virus (HIV).

A pharmaceutical composition for preventing and/or treating an immunodeficiency disease by immunostimulation may include the azabicyclo compound or a salt thereof; the azabicyclo compound or a salt thereof may be used for preventing and/or treating an immunodeficiency disease by immunostimulation; and a method for preventing and/or treating an immunodeficiency disease in a patient in need of the prevention and/or treatment, by immunostimulation, may include administering to the patient an effective amount of the azabicyclo compound or a salt thereof.

Specific examples of the diseases caused by age-related decline in immune function include pneumonia.

A pharmaceutical composition for preventing and/or treating a disease caused by age-related decline in immune function, by immunostimulation may include the azabicyclo compound or a salt thereof; the azabicyclo compound or a salt thereof may be used for preventing and/or treating a disease caused by age-related decline in immune function, by immunostimulation; and a method for preventing and/or treating a disease caused by age-related decline in immune function in a patient in need of the prevention and/or treatment, by immunostimulation, may include administering to the patient an effective amount of the azabicyclo compound or a salt thereof.

Specific examples of the virus-related tumors, i.e. tumors developed by viral infection, include Burkitt's lymphoma, hepatocellular cancer, cervical cancer, adult T-cell leukemia, Kaposi sarcoma and head and neck cancer.

A pharmaceutical composition for preventing and/or treating a virus-related tumor by immunostimulation may include the azabicyclo compound or a salt thereof; the azabicyclo compound or a salt thereof may be used for preventing and/or treating a virus-related tumor by immunostimulation; and a method for preventing and/or treating a virus-related tumor in a patient in need of the prevention and/or treatment, by immunostimulation, may include administering to the patient an effective amount of the azabicyclo compound or a salt thereof.

In the present invention, the dose per dosing day of the azabicyclo compound or a salt thereof for immunostimulation is preferably 50 to 200%, more preferably 87.5 to 112.5%, particularly preferably 100%, of the recommended dosage of the singly administered azabicyclo compound or salt thereof, from the viewpoint of immunostimulatory action. The recommended dosage in a human is preferably 80 to 320 mg/body/day, more preferably 140 to 180 mg/body/day, particularly preferably 160 mg/body/day.

In the present invention, active ingredients in the azabicyclo compound or a salt thereof and the immune checkpoint molecule regulator may be separated and formulated in a plurality of dosage forms according to the formulations or administration schedules of the active ingredients, or integrated and formulated in one dosage form (i.e. formulated as a combination preparation). The preparations may be produced and sold in one package suitable for combination administration, or produced and sold in separate packages.

The formulations of the antitumor agent and the immunostimulant according to the present invention are not particularly limited, and can be appropriately selected according to the treatment purpose, and specific examples thereof include oral preparations (tablets, coated tablets, powders, granules, capsules, solutions, etc.), injections, suppositories, patches and ointments.

In the case of the azabicyclo compound or a salt thereof, oral preparations are preferable.

In the case of the anti-PD-1 antibody, anti-PD-L1 antibody or anti-CTLA-4 antibody, the aforementioned formulations are used, with injections being preferable.

For both the azabicyclo compound or a salt thereof and the immune checkpoint molecule regulator, the antitumor agent according to the present invention can be normally prepared by a known method using pharmaceutical acceptable carriers depending on the formulations of the agents. Examples of the carriers include various carriers which are commonly used for normal pharmaceutical agents, e.g. excipients, binders, disintegrants, lubricants, diluents, solubilizers, suspending agents, tonicity agents, pH adjustors, buffers, stabilizers, coloring agents, flavor improving agents and odor improving agents.

The present invention also relates to an antitumor effect enhancing agent for enhancing the antitumor effect of an immune checkpoint molecule regulator on a cancer patient, the antitumor effect enhancing agent containing the azabicyclo compound or a salt thereof as an active ingredient. The antitumor effect enhancing agent has a preparation form identical to that of the aforementioned antitumor agent.

The present invention also relates to an antitumor effect enhancing agent for enhancing the antitumor effect of the azabicyclo compound or a salt thereof on a cancer patient, the antitumor effect enhancing agent containing an immune checkpoint molecule regulator as an active ingredient. The antitumor effect enhancing agent has a preparation form identical to that of the aforementioned antitumor agent.

The present invention also relates to an antitumor agent for treating a cancer patient given an immune checkpoint molecule regulator, the antitumor agent containing the azabicyclo compound or a salt thereof. The antitumor agent has the aforementioned preparation form.

The present invention also relates to an antitumor agent for treating a cancer patient given the azabicyclo compound or a salt thereof, the antitumor agent containing an immune checkpoint molecule regulator. The antitumor agent has the aforementioned preparation form.

The "treatment" includes postoperative adjuvant chemotherapy to be carried out for prevention of recurrence after surgical removal of tumor, and preoperative adjuvant chemotherapy to be carried out before surgical removal of tumor.

The present invention also relates to an antitumor agent containing the azabicyclo compound or a salt thereof, used in combination with an immune checkpoint molecule regulator for a cancer patient. The antitumor agent has the aforementioned preparation form.

The present invention also relates to an antitumor agent containing an immune checkpoint molecule regulator, used in combination with an antitumor agent containing the azabicyclo compound or a salt thereof for a cancer patient. The antitumor agent has the aforementioned preparation form.

A kit preparation may include an antitumor agent containing the azabicyclo compound or a salt thereof; and a written instruction which indicates that the azabicyclo compound or a salt thereof and an immune checkpoint molecule regulator are administered in combination to a cancer patient.

Here, the "written instruction" is not limited as long as the aforementioned doses are specified. The written instruction may be legally binding or non-binding, and is preferably one in which the aforementioned doses are recommended. Specific examples of the written instruction include attached documents and brochures. The kit preparation including a written instruction may be one in which a written instruction is printed or attached on a kit preparation package, or one in which a written instruction is enclosed together with an antitumor agent in a kit preparation package.

Examples

The present invention will now be described in further detail by way of examples, which should not be construed as limiting the present invention, and those skilled in the art can make many modifications within the technical concept of the present invention.

Example 1: Effect of combination of Compound 1 and anti-mouse PD-1 antibody in bowel cancer cell transplanted model

Mouse bowel cancer cell line MC38 was obtained from Dr. Yoshihiro Hayakawa (University of Toyama, Toyama, Japan), and cultured in a RPMI1640 (Sigma-Aldrich) medium (culture solution) containing 10% fetal bovine serum (FBS). MC38 was subcultured at a ratio of 1:3 to 1:5 once or twice a week in an incubator with 5% $CO_2$ at 37°C.

A cell suspension with a concentration of $2 \times 10^6$ cells/0.1 mL was transplanted subcutaneously in the vicinity of the right backmost rib bone of a 6-week-old C57BL/6JJcI mouse (CLEA Japan, Inc.).

The tumor was grown until reaching a tumor volume (TV) of 50 to 300 mm$^3$ after the transplantation. Degimatic Caliper was used for tumor diameter measurement. The major diameter and the minor diameter of the tumor were measured, and TV was calculated from the following equation.

TV (mm$^3$) = major diameter (mm) $\times$ minor diameter (mm) $\times$ minor diameter (mm)/2

By a stratified random allocation method using TV as an index, 6 animals were allocated to each group. The day when grouping (n=6) was performed was defined as Day 0.

The relative tumor volume (RTV) and the relative tumor volume change ratio (T/C) were calculated from TV.

RTV and T/C were calculated from the following equation.

$$\texttt{RTV = TV on measurement day/TV on grouping day}$$

T/C (%) = (average RTV of dosed group)/average RTV of control group) $\times$ 100

An electronic balance for animals was used for body weight measurement. The body weight change ratio on the nth day (BWCn) was calculated from the body weight on the nth day (BWn) in accordance with the following equation.

$$\texttt{Body weight change ratio BWCn (\%) = (BWn-BW0)/BW0} \times \texttt{100}$$

An appropriate amount of water for injection described in the Japanese Pharmacopeia was added for adjusting hypromellose concentration to 0.5 w/v%, and the mixture was then stirred with a stirrer to completely dissolve hypromellose, to prepare a 0.5% aqueous hypromellose solution. Compound 1 was ground with an agate mortar, and then suspended to a predetermined concentration with the 0.5% aqueous hypromellose solution, and ultrasonic treatment was performed to obtain a homogeneous suspension. The suspension containing Compound 1 was orally administered once a day over 5 consecutive days at a dose of 10 mg/kg/day or 14 mg/kg/day, followed by cessation of administration for 2 days, and these processes were repeated three times.

The anti-mouse PD-1 antibody (anti-mPD-1 Ab) was prepared by diluting InVivoMab anti-mouse PD-1 (CD279) (Clone: RMP-1-14, Bio X cell Co.) to a predetermined concentration with Otsuka Normal Saline immediately before administration. On the first day of administration (day 1), the diluted product was intraperitoneally administered as the anti-mouse PD-1 antibody at a dose of 0.05 mg/body.

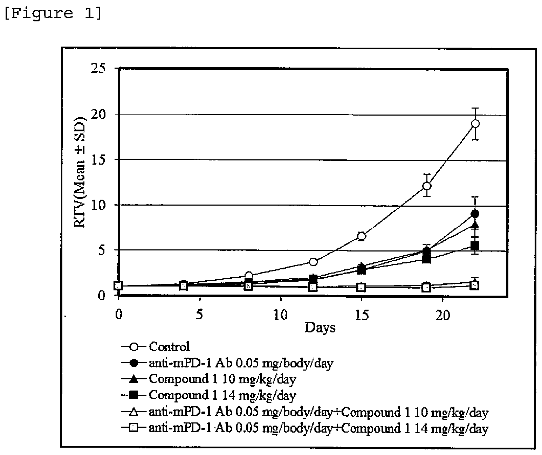

The results are shown in Figure 1 and Table 1.

[Table 1]

| Group | Dose (/day) | Schedule (day) | Route | RTV Day 22 (mean ± SD) | | T/C (%) | BWC(%) Day 22 Mean |
|---|---|---|---|---|---|---|---|
| Control | - | 1-5, 8-12, 15-19 | p.o. | 19.02 ± 1.77 | | 100.0 | 11.8 |
| Anti-mPD-1 Ab | 0.05 mg/body | 1 | i.p. | 9.18 ± 1.85 | ** | 48.3 | 15.5 |
| Compound 1 | 10 mg/kg | 1-5, 8-12, 15-19 | p.o. | 7.93 ± 1.29 | ** | 41.7 | 13.5 |
| Compound 1 | 14 mg/kg | 1-5, 8-12, 15-19 | p.o. | 5.59 ± 0.92 | ** | 29.4 | 12.8 |
| Anti-mPD-1 Ab +Compound 1 | 0.05 mg/body +10 mg/kg | 1 +1-5, 8-12, 15-19 | i.p +p.o. | 1.58 ± 0.55 | **##$$ | 8.3 | 12.7 |

(continued)

| Group | Dose (/day) | Schedule (day) | Route | RTV | | T/C (%) | BWC(%) |
| | | | | Day 22 | | | Day 22 |
| | | | | (mean ± SD) | | | Mean |
| --- | --- | --- | --- | --- | --- | --- | --- |
| Anti-mPD-1 Ab +Compound 1 | 0.05 mg/body +14 mg/kg | 1 +1-5, 8-12, 15-19 | ip. +p.o. | 1.16 ± 0.41 | **##$$ | 6.1 | 15.1 |

\*\*: p<0.01 with Aspin-Welch's *t*- test as compared with the Control group.
\#\#: p<0.01 with Aspin-Welch's *t*- test as compared with the anti-mouse PD-1 antibody (anti-mPD-1 Ab) group.
\$\$: p<0.01 with Aspin-Welch's *t*- test as compared with the Compound 1 group.
SD: standard deviation

RTV of each group on Day 22 was analyzed by the Aspin-Welch's t-test, and the result showed that both the single-agent group to which Compound 1 group or the anti-mouse PD-1 antibody was administered, and the combination administration group to which Compound 1 + anti-mouse PD-1 antibody were administered exhibited an antitumor effect with significantly lower RTV as compared to the control group. Further, the combination administration group to which Compound 1 + anti-mouse PD-1 antibody were administered had significantly lower RTV and exhibited a higher antitumor effect as compared to the single-agent group to which Compound 1 or the anti-mouse PD-1 antibody was administered.
The average body weight change ratio of the combination administration group indicated no enhanced toxicity as compared to the single-agent group to which Compound 1 or the anti-mouse PD-1 antibody alone was administered.

Example 2: Immunostimulatory action of Compound 1 in bowel cancer cell transplanted model

Mouse bowel cancer cell line MC38 was cultured using a RPMI1640 (Waco Pure Chemical Industries, Ltd.) medium containing 10% FBS. MC38 was subcultured at a ratio of 1:5 to 1:10 once or twice a week in an incubator with 5% $CO_2$ at 37°C.
A cell suspension with a concentration of $2\times10^6$ cells/0.1 mL was transplanted subcutaneously in the vicinity of the right backmost rib bone of a 6-week-old C57BL/6NJCrI mouse (Charles River Laboratories Japan Inc.).
The tumor was grown until reaching a tumor volume (TV) of 110 to 360 $mm^3$ after the transplantation. Degimatic Caliper was used for tumor diameter measurement. The major diameter and the minor diameter of the tumor were measured, and TV was calculated from the following equation.

TV ($mm^3$) = major diameter (mm) $\times$ minor diameter (mm) $\times$ minor diameter (mm)/2

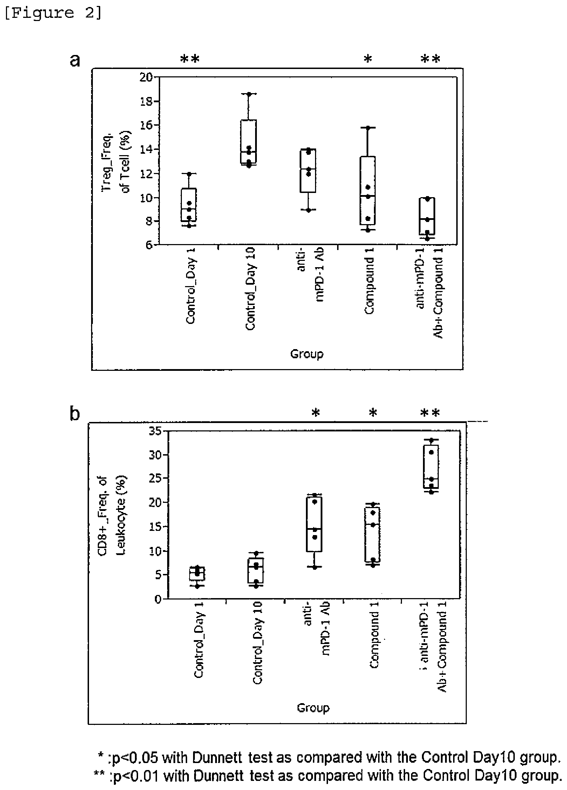

By a stratified random allocation method using TV as an index, 5 animals were allocated to each group. The day when grouping (n=5) was performed was defined as Day 1.
Agents prepared by the same method as described in Example 1 were used for administration. Anti-mouse PD-1 antibody (anti-mPD-1 Ab), CD279(PD-1)Monoclonal Antibody (RMP1-14), eBioscience (Thermo Fisher Scientific), was used as an anti-mouse PD-1 antibody, and intraperitoneally administered at a dose of 0.05 mg/body on the first day of administration (Day 1). Compound 1 was orally administered at a dose of 14 mg/kg/day once a day on Days 1-5 and 8-9.
For detecting expression of immunity-related factors, flow cytometric analysis was performed for immunological monitoring. On Day 10, the tumor was sampled, and tumor infiltrating lymphocytes were then prepared using Tumor Dissociation Kit, mouse (Miltenyi Biotec). Thereafter, the antibody was stained using FoxP3/Transcription Factor Staining Buffer Set (thermo Fisher Scientific), and analysis was then performed using Flow Cytometer FACSVerse (BD Bioscience). For the staining, CD45 Monoclonal Antibody (30-F11), eFluor 450, eBioscience™ (Thermo Fisher Scientific), FoxP3 Monoclonal Antibody (FJK-16s), PE, eBioscience ™ (Thermo Fisher Scientific), CD4 Monoclonal Antibody (GK1.5), PE-Cyanine7, eBioscienceTM (Thermo Fisher Scientific) and CD25 Monoclonal Antibody (PC61.5), APC, eBioscience™ (Thermo Fisher Scientific), Brilliant Violet 510™ anti-mouse CD90.2 Antibody (BioLegend) and CD8a MonoClonal Antibody (53-6.7), FITC, eBioscience™ (BioLegend) were used.
The results are shown in Figure 2.
The ratio of CD8 positive cells (CD45 positive, CD90.2 positive and CD8 positive cells) in leucocytes (CD45 positive cells) of each group on Day 10 was analyzed by the Dunnett test, and the result showed that the Compound 1-administered group had a significantly higher ratio of CD8 positive cells in leucocytes as compared to the control group on Day 10. The ratio of regulatory T-cells (CD45 positive, CD90.2 positive, CD4 positive, CD25 positive and FoxP3 positive cells) in T-cells (CD45 positive and CD90.2 positive cells) of each group on Day 10 was analyzed by the Dunnett test, and the result showed that the Compound 1-administered group had a significantly lower ratio of regulatory T-cells in T-cells as compared to the control group on Day 10.

The above results showed that Compound 1 shows immunostimulatory activity.

Example 3: Effect of combination of Compound 1 and anti-mouse PD-L1 antibody or anti-mouse CTLA-4 antibody in bowel cancer cell transplanted model

The same procedure as described in Example 1 was carried out except that the anti-mouse PD-L1 antibody or the anti-CTLA-4 antibody was used instead of the anti-mouse PD-1 antibody.

The anti-mouse PD-L1 antibody (anti-mPD-L1 Ab) was prepared by diluting InVivoMab anti-mouse PD-L1 (B7-H1) (Clone: 10F.9G2, Bio X cell Co.) to a predetermined concentration with Otsuka Normal Saline immediately before administration. On the first day of administration (day 1), the diluted product was intraperitoneally administered using the anti-mouse PD-L1 antibody at a dose of 0.05 mg/body. The anti-mouse CTLA-4 antibody (anti-mCTLA-4 Ab) was prepared by diluting InVivoMab anti-mouse CTLA-4 (CD152) (Clone: 9H10, Bio X cell Co.) to a predetermined concentration with Otsuka Normal Saline immediately before administration. On the first day of administration (day 1), the diluted product was intraperitoneally administered using the anti-mouse CTLA-4 antibody at a dose of 0.05 mg/body.

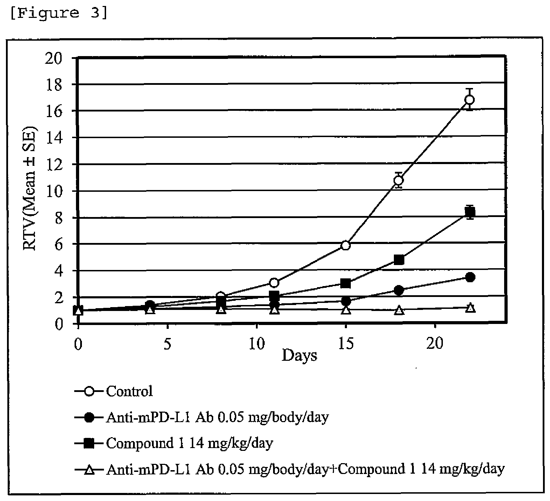

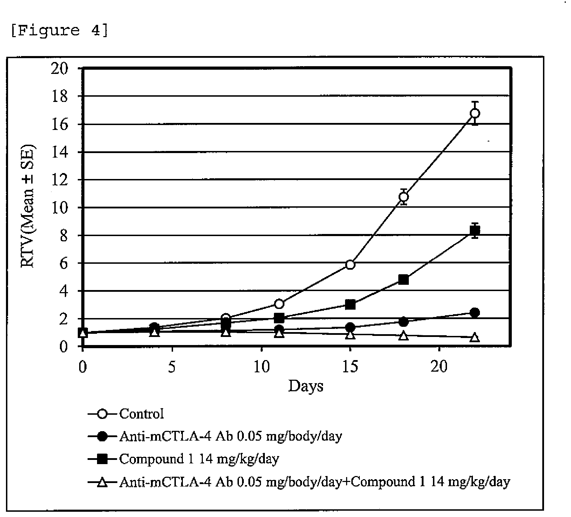

The results are shown in Figures 3 and 4 and Table 2.

[Table 2]

| Group | Dose (/day) | Schedule (day) | Route | RTV Day 22 (mean ± SE) | | T/C (%) | BWC(%) Day 22 Mean |
|---|---|---|---|---|---|---|---|
| Control | - | 1-5, 8-12, 15-19 | p.o. | 16.75 ± 0.81 | | 100.0 | 5.88 |
| Anti-mPD-L1 Ab | 0.05 mg/body | 1 | i.p. | 3.43 ± 0.25 | ** | 20.5 | 7.95 |
| Anti-mCTLA-4 Ab | 0.05 mg/body | 1 | i.p. | 2.41 ± 0.21 | ** | 14.4 | 7.17 |
| Compound 1 | 14 mg/kg | 1-5, 8-12, 15-19 | p.o. | 8.32 ± 0.51 | ** | 49.7 | 6.63 |
| Anti-mPD-L1 Ab +Compound 1 | 0.05 mg/body +14 mg/kg | 1 +1-5, 8-12, 15-19 | i.p +p.o. | 1.14 ± 0.13 | **##$$ | 6.8 | 7.01 |
| Anti-mCTLA-4 Ab +Compound 1 | 0.05 mg/body +14 mg/kg | 1 +1-5, 8-12, 15-19 | i.p. +p.o. | 0.65 ± 0.03 | **&&$$ | 3.9 | 5.97 |

**: $p < 0.01$ with Aspin-Welch's $t$-test as compared with the Control group.
##: $p < 0.01$ with Aspin-Welch's $t$-test as compared with the anti-mouse PD-L1 antibody (anti-mPD-L1 Ab) group.
&&: $p < 0.01$ with Aspin-Welch's $t$-test as compared with the anti-mouse CTLA-4 antibody (anti-mCTLA-4 Ab) group.
$$: $p < 0.01$ with Aspin-Welch's $t$-test as compared with the Compound 1 group.
SE: standard error

RTV of each group on Day 22 was analyzed by the Aspin-Welch's t-test, and the result showed that the single-agent group to which Compound 1, the anti-mouse PD-L1 antibody or the anti-mouse CTLA-4 antibody was administered, and the combination administration group to which Compound 1 + anti-mouse PD-L1 antibody or the anti-mouse CTLA-4 antibody were administered exhibited an antitumor effect with significantly lower RTV as compared to the control group. Further, the combination administration group to which Compound 1 + anti-mouse PD-L1 antibody were administered had significantly lower RTV and exhibited a higher antitumor effect as compared to the single-agent group to which Compound 1 or the anti-mouse PD-L1 antibody was administered. The combination administration group to which Compound 1 + anti-mouse CTLA-4 antibody were administered had significantly lower RTV and exhibited a higher antitumor effect as compared to the single-agent group to which Compound 1 or the anti-mouse CTLA-4 antibody was administered.

The average body weight change ratio of the combined administration group indicated no enhanced toxicity as compared to the single-agent group to which Compound 1, the anti-mouse PD-L1 antibody or the anti-mouse CTLA-4 antibody alone was administered.

**Claims**

1. An antitumor agent comprising azabicyclo compound or a salt thereof and an immune checkpoint molecule regulator which are administered in combination, wherein the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide;

wherein the immune checkpoint molecule regulator is at least one or more selected from the group consisting of a PD-1 pathway antagonist and a CTLA-4 pathway antagonist,
wherein the PD-1 pathway antagonist is at least one or more selected from the group consisting of an anti-PD-1 antibody and an anti-PD-L1 antibody, and
wherein the CTLA-4 pathway antagonist is an anti-CTLA-4 antibody.

2. The antitumor agent according to claim 1, wherein the anti-PD-1 antibody is at least one or more selected from the group consisting of nivolumab and pembrolizumab, and the anti-PD-L1 antibody is at least one or more selected from the group consisting of atezolizumab, durvalumab and avelumab.

3. The antitumor agent according to claim 1 or 2, wherein the anti-CTLA-4 antibody is at least one or more selected from the group consisting of ipilimumab and tremelimumab.

4. An agent that is

(i) an antitumor effect enhancing agent for use in enhancing the antitumor effect of an immune checkpoint molecule regulator in tumor treatment, comprising an azabicyclo compound or a salt thereof as an active ingredient, or
(ii) an antitumor agent for use in treating a cancer patient given an immune checkpoint molecule regulator, wherein both are administered in parallel or at an administration interval of 1 to 14 days, the antitumor agent comprising an azabicyclo compound or a salt thereof, or
(iii) an antitumor agent for use in treating a cancer patient given an azabicyclo compound or a salt thereof, wherein both are administered in parallel or at an administration interval of 1 to 14 days, the antitumor agent comprising an immune checkpoint molecule regulator;
wherein the azabicyclo compound is as defined in claim 1 and the immune checkpoint molecule regulator is as defined in any one of claims 1 to 3.

5. An antitumor agent comprising an azabicyclo compound or a salt thereof in combination with an immune checkpoint molecule regulator; wherein the azabicyclo compound is as defined in claim 1 and the immune checkpoint molecule regulator is as defined in any one of claims 1 to 3.

6. An azabicyclo compound or a salt thereof for use in treating a tumor by administration thereof in combination with an immune checkpoint molecule regulator, the azabicyclo compound being as defined in claim 1 and the immune checkpoint molecule regulator being as defined as in any one of claims 1 to 3.

7. An azabicyclo compound or a salt thereof for use

(i) in enhancing the antitumor effect of an immune checkpoint molecule regulator, or
(ii) in treating tumor in a cancer patient given an immune checkpoint molecule regulator in parallel or at an administration interval of 1 to 14 days,
the azabicyclo compound being as defined in claim 1 and the immune checkpoint molecule regulator is as defined in any one of claims 1 to 3.

8. An immune checkpoint molecule regulator for use in treating a tumor in a cancer patient given an azabicyclo compound or a salt thereof, wherein both are administered in parallel or at an administration interval of 1 to 14 days, the azabicyclo compound being as defined in claim 1 and the immune checkpoint molecule regulator is as defined in any one of claims 1 to 3.

9. A combination of an azabicyclo compound or a salt thereof and an immune checkpoint molecule regulator for use in treating a tumor, wherein both are administered in parallel or at an administration interval of 1 to 14 days, the azabicyclo compound being as defined in claim 1 and the immune checkpoint molecule regulator is as defined in any one of claims 1 to 3.

**Patentansprüche**

1. Antitumormittel, umfassend eine Azabicyclo-Verbindung oder ein Salz davon und einen Immun-Checkpoint-Molekül-Regulator, die in Kombination verabreicht werden, wobei die Azabicyclo-Verbindung 3-Ethyl-4-{3-isopropyl-4-(4-(1-methyl-1H-pyrazol-4-yl)-1H-imidazol-1-yl)-1H-pyrazolo[3,4-b]pyridin-1-yl}benzamid ist;

   wobei der Immun-Checkpoint-Molekül-Regulator mindestens einer oder mehrere ist, die aus der Gruppe ausgewählt sind, die aus einem PD-1-Pathway-Antagonisten und einem CTLA-4-Pathway-Antagonisten besteht, wobei der PD-1-Signalweg-Antagonist mindestens einer oder mehrere ist, die aus der Gruppe ausgewählt sind, die aus einem Anti-PD-1-Antikörper und einem Anti-PD-L1-Antikörper besteht, und wobei der CTLA-4-Pathway-Antagonist ein Anti-CTLA-4-Antikörper ist.

2. Das Antitumormittel nach Anspruch 1, wobei der Anti-PD-1-Antikörper mindestens einer oder mehrere aus der Gruppe bestehend aus Nivolumab und Pembrolizumab ist und der Anti-PD-L1-Antikörper mindestens einer oder mehrere aus der Gruppe bestehend aus Atezolizumab, Durvalumab und Avelumab ist.

3. Das Antitumormittel nach Anspruch 1 oder 2, wobei es sich bei dem Anti-CTLA-4-Antikörper um mindestens einen oder mehrere Antikörper handelt, die aus der Gruppe bestehend aus Ipilimumab und Tremelimumab ausgewählt sind.

4. Ein Mittel, das

   (i) ein die Antitumorwirkung verstärkendes Mittel zur Verwendung bei der Verstärkung der Antitumorwirkung eines Immun-Checkpoint-Molekül-Regulators bei der Tumorbehandlung, umfassend eine Azabicycloverbindung oder ein Salz davon als Wirkstoff, oder
   (ii) ein Antitumormittel zur Verwendung bei der Behandlung eines Krebspatienten, dem ein Immun-Checkpoint-Molekül-Regulator verabreicht wurde, wobei beide parallel oder in einem Verabreichungsintervall von 1 bis 14 Tagen verabreicht werden, wobei das Antitumormittel eine Azabicycloverbindung oder ein Salz davon umfasst, oder
   (iii) ein Antitumormittel zur Verwendung bei der Behandlung eines Krebspatienten, dem eine Azabicycloverbindung oder ein Salz davon verabreicht wird, wobei beide parallel oder in einem Verabreichungsintervall von 1 bis 14 Tagen verabreicht werden, wobei das Antitumormittel einen Immun-Checkpoint-Molekül-Regulator umfasst;
   worin die Azabicycloverbindung wie in Anspruch 1 definiert ist und der Immun-Checkpoint-Molekül-Regulator wie in einem der Ansprüche 1 bis 3 definiert ist.

5. Antitumormittel, umfassend eine Azabicyclo-Verbindung oder ein Salz davon in Kombination mit einem Immun-Checkpoint-Molekül-Regulator; wobei die Azabicyclo-Verbindung wie in Anspruch 1 definiert ist und der Immun-Checkpoint-Molekül-Regulator wie in einem der Ansprüche 1 bis 3 definiert ist.

6. Azabicycloverbindung oder ein Salz davon zur Verwendung bei der Behandlung eines Tumors durch Verabreichung davon in Kombination mit einem Immun-Checkpoint-Molekül-Regulator, wobei die Azabicycloverbindung wie in Anspruch 1 definiert ist und der Immun-Checkpoint-Molekül-Regulator wie in einem der Ansprüche 1 bis 3 definiert ist.

7. Azabicyclo-Verbindung oder ein Salz davon zur Verwendung

   (i) bei der Verstärkung der Antitumorwirkung eines Immun-Checkpoint-Molekül-Regulators, oder
   (ii) bei der Behandlung eines Tumors bei einem Krebspatienten, dem ein Immun-Checkpoint-Molekül-Regulator parallel oder in einem Verabreichungsintervall von 1 bis 14 Tagen verabreicht wird,
   wobei die Azabicycloverbindung wie in Anspruch 1 definiert ist und der Immun-Checkpoint-Molekül-Regulator wie in einem der Ansprüche 1 bis 3 definiert ist.

8. Immun-Checkpoint-Molekül-Regulator zur Verwendung bei der Behandlung eines Tumors in einem Krebspatienten, dem eine Azabicyclo-Verbindung oder ein Salz davon verabreicht wird, wobei beide parallel oder in einem Verabreichungsintervall von 1 bis 14 Tagen verabreicht werden, wobei die Azabicyclo-Verbindung wie in Anspruch 1 definiert ist und der Immun-Checkpoint-Molekül-Regulator wie in einem der Ansprüche 1 bis 3 definiert ist.

9. Kombination aus einer Azabicyclo-Verbindung oder einem Salz davon und einem Immun-Checkpoint-Molekül-Regulator zur Verwendung bei der Behandlung eines Tumors, wobei beide parallel oder in einem Verabreichungsintervall von 1 bis 14 Tagen verabreicht werden, wobei die Azabicyclo-Verbindung wie in Anspruch 1 definiert ist und der Immun-Checkpoint-Molekül-Regulator wie in einem der Ansprüche 1 bis 3 definiert ist.

**Revendications**

1. Agent antitumoral comprenant un composé azabicyclo ou un sel de celui-ci et un régulateur de molécule de point de contrôle immunitaire qui sont administrés en combinaison, dans lequel le composé azabicyclo est le 3-éthyl-4-{3-isopropyl-4-(4-(1-méthyl-1H-pyrazol-4-yl)-1H-imidazol-1-yl)-1H-pyrazolo[3,4-b]pyridin-1-yl}benzamide ;

   dans lequel le régulateur de molécule de point de contrôle immunitaire est au moins un ou plusieurs éléments choisis dans le groupe consistant en un antagoniste de la voie PD-1 et un antagoniste de la voie CTLA-4, dans lequel l'antagoniste de la voie PD-1 est au moins un ou plusieurs éléments choisis dans le groupe consistant en un anticorps anti-PD-1 et un anticorps anti-PD-L1, et dans lequel l'antagoniste de la voie CTLA-4 est un anticorps anti-CTLA-4.

2. Agent antitumoral selon la revendication 1, dans lequel l'anticorps anti-PD-1 est au moins un ou plusieurs éléments choisis dans le groupe consistant en nivolumab et pembrolizumab, et l'anticorps anti-PD-L1 est au moins un ou plusieurs éléments choisis dans le groupe consistant en atézolizumab, durvalumab et avélumab.

3. Agent antitumoral selon la revendication 1 ou 2, dans lequel l'anticorps anti-CTLA-4 est au moins un ou plusieurs éléments choisis dans le groupe consistant en ipilimumab et trémélimumab.

4. Agent qui est

   (i) un agent amplifiant l'effet antitumoral à utiliser pour amplifier l'effet antitumoral d'un régulateur de molécule de point de contrôle immunitaire dans le traitement de tumeur, comprenant un composé azabicyclo ou un sel de celui-ci en tant qu'ingrédient actif, ou
   (ii) un agent antitumoral à utiliser dans le traitement d'un patient cancéreux se voyant donner un régulateur de molécule de point de contrôle immunitaire, dans lequel les deux sont administrés en parallèle ou à un intervalle d'administration de 1 à 14 jours, l'agent antitumoral comprenant un composé azabicyclo ou un sel de celui-ci, ou
   (iii) un agent antitumoral à utiliser dans le traitement d'un patient cancéreux se voyant donner un composé azabicyclo ou un sel de celui-ci, dans lequel les deux sont administrés en parallèle ou à un intervalle d'administration de 1 à 14 jours, l'agent antitumoral comprenant un régulateur de molécule de point de contrôle immunitaire ;

   dans lequel le composé azabicyclo est tel que défini dans la revendication 1 et le régulateur de molécule de point de contrôle immunitaire est tel que défini dans l'une des revendications 1 à 3.

5. Agent antitumoral comprenant un composé azabicyclo ou un sel de celui-ci en combinaison avec un régulateur de molécule de point de contrôle immunitaire ; dans lequel le composé azabicyclo est tel que défini dans la revendication 1 et le régulateur de molécule de point de contrôle immunitaire est tel que défini dans l'une des revendications 1 à 3.

6. Composé d'azabicyclo ou sel de celui-ci à utiliser dans le traitement d'une tumeur par administration de celui-ci en combinaison avec un régulateur de molécule de point de contrôle immunitaire, le composé d'azabicyclo étant tel que défini dans la revendication 1 et le régulateur de molécule de point de contrôle immunitaire étant tel que défini dans l'une quelconque des revendications 1 à 3.

7. Composé azabicyclo ou sel de celui-ci à utiliser

   (i) dans l'amplification de l'effet antitumoral d'un régulateur de molécule de point de contrôle immunitaire, ou
   (ii) dans le traitement d'une tumeur chez un patient cancéreux se voyant donner un régulateur de molécule de point de contrôle immunitaire en parallèle ou à un intervalle d'administration de 1 à 14 jours,

   le composé azabicyclo étant tel que défini dans la revendication 1 et le régulateur de molécule de point de contrôle immunitaire étant tel que défini dans l'une des revendications 1 à 3.

8. Régulateur de molécule de point de contrôle immunitaire à utiliser dans le traitement d'une tumeur chez un patient cancéreux se voyant donner un composé azabicyclo ou un sel de celui-ci, dans lequel les deux sont administrés en parallèle ou à un intervalle d'administration de 1 à 14 jours, le composé azabicyclo étant tel que défini dans la revendication 1 et le régulateur de molécule de point de contrôle immunitaire étant tel que défini dans l'une des revendications 1 à 3.

9. Combinaison d'un composé azabicyclo ou d'un sel de celui-ci et d'un régulateur de molécule de point de contrôle immunitaire à utiliser dans le traitement d'une tumeur, dans laquelle les deux sont administrés en parallèle ou à un intervalle d'administration de 1 à 14 jours, le composé azabicyclo étant tel que défini dans la revendication 1 et le régulateur de molécule de point de contrôle immunitaire étant tel que défini dans l'une des revendications 1 à 3.

[Figure 1]

[Figure 2]

* :p<0.05 with Dunnett test as compared with the Control Day10 group.
** :p<0.01 with Dunnett test as compared with the Control Day10 group.

[Figure 3]

[Figure 4]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004004771 A
- WO 2011004610 A
- WO 2015046498 A
- WO 2016130502 A

**Non-patent literature cited in the description**

- *Nat. Rev. Cancer*, 2005, vol. 5, 761-772
- *TRENDS Mol. Med.*, 2004, vol. 10, 283-290
- *Clin. Cancer Res.*, 2009, vol. 15, 9-14
- *Curr. Opin. Pharmacol.*, 2008, vol. 8, 370-374
- *Drug Resist. Updat.*, 2009, vol. 12, 17-27
- *Nat. Rev. Cancer*, 2012, vol. 12, 252-264
- *N. Engl. J. Med.*, 2012, vol. 366, 2443-2454
- **MBOFUNG RINA M et al.** Mechanistic Merging of Treatment Modalities. *Cancer Immunology Research*, 01 November 2016
- *Nat. Rev. Immunol.*, 2006, vol. 6, 343-357